# EUROPÄISCHE PATENTSCHRIFT

(11) **EP 0 551 838 B1**
(45) Veröffentlichungstag und Bekanntmachung des Hinweises auf die Patenterteilung: **25.09.1996**
(21) Anmeldenummer: 93100236.4
(22) Anmeldetag: 09.01.1993
(51) Int. Cl.: C01B 7/19, C07C 17/38

(54) **Aufarbeitung der Fluorwasserstoff-Phase aus Reaktionsgemischen**
Treatment of the hydrogen fluoride-phase from reaction mixtures
Traitement d'une phase d'acide fluorhydrique des mélanges réactionnels

(30) Priorität: 15.01.1992 DE 4200792
(43) Veröffentlichungstag der Anmeldung: 21.07.1993
(73) Patentinhaber: Solvay Fluor und Derivate GmbH, D-30173 Hannover (DE)
(72) Erfinder: Eicher, Johannes, W-3008 Garbsen 1 (DE); Rudolph, Werner, W-3000 Hannover 71 (DE); Schulte, Bernhard, B-1933 Sterrebeck (BE)
(74) Vertreter: Lauer, Dieter, Dr.

(56) Entgegenhaltungen:
- EP-A- 0 353 970
- EP-A- 0 445 560
- WO-A-82/03848
- NL-A- 8 502 333
- ULLMANN'S ENCYKLOPÄDIE DER TECHNISCHEN CHEMIE, Band 21, Seiten 161-162

## Beschreibung

Die Erfindung bezieht sich auf ein Verfahren zur Aufarbeitung der Fluorwasserstoff-Phase von Reaktionsgemischen aus Fluorierungsreaktionen.

Fluor enthaltende Kohlenwasserstoffe werden in großem Umfang hergestellt, indem Halogenkohlenwasserstoffe mit Fluorwasserstoff umgesetzt werden. Gewöhnlich geht man von Chlor enthaltenden Kohlenwasserstoffen aus. Neben vollhalogenierten Kohlenstoff-Verbindungen können natürlich auch Verbindungen eingesetzt bzw. hergestellt werden, die neben Fluor (und gegebenenfalls Chlor) auch Wasserstoff enthalten. Zu ihrer Herstellung kann man z.B. auch Alkene einsetzen, so daß auch eine Fluorwasserstoff-Addition erfolgt. Verbindungen dieser Art haben als Ersatzstoffe für vollhalogenierte Kohlenstoff-Verbindungen Bedeutung erlangt. Die DE-OS 40 05 945 beschreibt ein solches Verfahren. Aus halogenhaltigen Alkenen oder Alkanen werden unter Halogen-Fluor-Austausch und gegebenenfalls Fluorwasserstoff-Addition trifluormethylgruppenhaltige Ethanderivate, z.B. CF₃CHCl₂, hergestellt. Im Verlauf der Aufarbeitung werden flüchtige Reaktionsprodukte aus dem Reaktionsgemisch zunächst in einem Phasentrenner gesammelt. Es scheiden sich dabei zwei Phasen ab. Die schwere Phase enthält überwiegend organische Verbindungen, insbesondere das gewünschte Reaktionsprodukt. Die leichtere Phase enthält überwiegend Fluorwasserstoff und Wasser. Die Fluorwasserstoff-Phase wird in die Fluorierungsreaktion rückgeführt, im Phasentrenner etwaig freigesetzte leichtflüchtige Bestandteile werden durch einen Gaswäscher geleitet. Nachteil dieses Verfahrens ist, daß das in der Fluorwasserstoff-Phase enthaltene Wasser im Kreislauf geführt wird.

Die EP-A-0 353 970 offenbart, daß man Gemische, welche Fluorwasserstoff und Fluorverbindungen (Difluortrichlorethan bzw. Tetrafluorchlorethan) enthalten, durch Phasentrennung vorseparieren kann, worauf die erhaltene HF- bzw. organische Phase durch Destillation weiter gereinigt werden kann.

Die niederländische Patentanmeldung NL-A 85 02 333 betrifft ein Verfahren zur Rückgewinnung von Fluorwasserstoff aus Gemischen, die Fluorwasserstoff, Schwefelsäure und Wasser enthalten. Dieses Gemisch wird mit einem Gemisch aus Fluorwasserstoff und Wasserdampf kontaktiert; das daraus resultierende Dampfgemisch wird mit konzentrierter Schwefelsäure bei einer Temperatur von 70 bis 100°C behandelt. Es resultiert ein Dampf, der aus Fluorwasserstoff und wenig Wasser besteht, sowie verdünnte Schwefelsäure, die nur noch wenig Fluorwasserstoff enthält.

Die vorliegende Erfindung hat sich zur Aufgabe gesetzt, ein Verfahren anzugeben, mit welchem die Verwendbarkeit der von der organischen Phase abgetrennten Fluorwasserstoff-Phase verbessert wird. Diese Aufgabe wird gelöst durch das in den Ansprüchen wiedergegebene erfindungsgemäße Verfahren zur Aufarbeitung der Fluorwasserstoff-Phase von Reaktionsgemischen aus Fluorierungsreaktionen, wobei man aus der Reaktionsmischung zwei Phasen bildet, deren eine Phase im wesentlichen aus organischem Reaktionsprodukt besteht und die andere Phase eine Fluorwasserstoff-Phase ist, man die Fluorwasserstoff-Phase von der anderen Phase abtrennt, man die Fluorwasserstoff-Phase zur Wasserabtrennung bei einer Temperatur von 160°C bis 200°C mit Oleum kontaktiert und den resultierenden Fluorwasserstoff in die Fluorierungsreaktion rückführt.

Das Kontaktieren erfolgt vorzugsweise in einer Füllkörperkolonne im Gegenstrom, kann aber auch durch Ausschütteln oder Durchleiten durch Oleum erfolgen.

Gewünschtenfalls kann man den Fluorwasserstoff nach dem Kontaktieren mit Oleum weiter fraktionieren, um mitgerissene Schwefelsäure oder Oleum abzutrennen. Dieses kann in einer separaten Rektifikationskolonne erfolgen. Zweckmäßig verwendet man jedoch eine Füllkörperkolonne mit aufgesetzter Rektifikationskolonne. Die Fluorwasserstoff-Phase wird dabei in die Füllkörperkolonne eingespeist und in der Hitze bei Temperaturen von 160°C bis 200°C, mit Oleum getrocknet. Die heißen Fluorwasserstoff-Gase, die mit mitgerissener Schwefelsäure oder Oleum verunreinigt sein können, steigen in die Rektifikationskolonne auf. Reiner Fluorwasserstoff wird über Kopf abgezogen, Schwefelsaure und andere Schwersieder werden abgeschieden.

Die über Kopf abgezogene HF-Fraktion kann noch HCl und niedrigsiedende organische Bestandteile enthalten und kann unmittelbar rückgeführt werden.

Die abgezogene Fluorwasserstoff-Fraktion enthält, wenn überhaupt, nur geringe Mengen an Wasser.

Das verwendete Oleum kann mehr oder weniger SO₃ enthalten. Zweckmäßig verwendet man Oleum mit einem SO₃-Gehalt zwischen 1 und 40 Gew.-%, bezogen auf die als 100 Gew.-% gesetzte H₂SO₄.

Das bei der Trocknung verbrauchte Oleum kann durch Versetzen mit SO₃ und/oder Oleum wieder aufgestärkt werden. Man kann das verbrauchte Oleum aber auch in einem Verfahren zur Herstellung von Fluorwasserstoff durch Umsetzung von Flußspat (CaF₂) mit Schwefelsäure bzw. Oleum verwenden. Bekanntermaßen kann Fluorwasserstoff dadurch hergestellt werden, daß Flußspat mit Schwefelsäure oder Oleum unter Bildung von Calciumsulfat und Fluorwasserstoff umgesetzt wird. Dies erfolgt gewöhnlich bei sehr hoher Temperatur (200 °C und mehr) in Drehöfen. Gemäß einer bevorzugten Ausführungsform des erfindungsgemäßen Verfahrens wird das mit Wasser beladene Oleum bzw. die dabei resultierende Schwefelsäure verwendet, um einen Teil der zur Umsetzung von Flußspat mit Schwefelsäure benötigten Schwefelsäure bzw. des benötigten Oleums zu ersetzen.

Natürlich kann man auch einen Teil des aus der Trocknung des Fluorwasserstoffs resultierenden verbrauchten Oleums unter Aufstärkung mit SO₃ und/oder Oleum im Kreislauf führen, und der andere Teil wird bei der Fluorwasserstoff-Herstellung aus Calciumfluorid verwendet.

Der Druck in der Rektifikationskolonne liegt zweckmäßig im Bereich von 1 bis 2 bar, vorzugsweise im Bereich von 1,5 bis 2 bar.

Die Rektifikationskolonne wird zweckmäßig so temperiert, daß sie im oberen Bereich (Kolonnenkopf) eine Temperatur von 10 bis 20 °C und im unteren Bereich eine Temperatur von 160 bis 200 °C aufweist.

Das erfindungsgemäße Aufarbeitungsverfahren wird im Rahmen von Verfahren zur Herstellung von Fluor und gegebenenfalls anderes Halogen enthaltenden Kohlenwasserstoffen durchgeführt. Solche Verfahren zur Herstellung von Fluor enthaltenden Kohlenwasserstoffen umfassen einen Halogen-Fluor-Austausch unter Verwendung von überschüssigem Fluorwasserstoff, Aufarbeiten der Reaktionsmischung unter Bildung zweier Phasen, deren eine Phase im wesentlichen aus organischen Verbindungen besteht und deren andere Phase eine Fluorwasserstoff-Phase ist, Abtrennen der Fluorwasserstoff-Phase von der anderen Phase und Aufarbeiten und Rückführen der Fluorwasserstoff-Phase nach dem erfindungsgemäßen Verfahren.

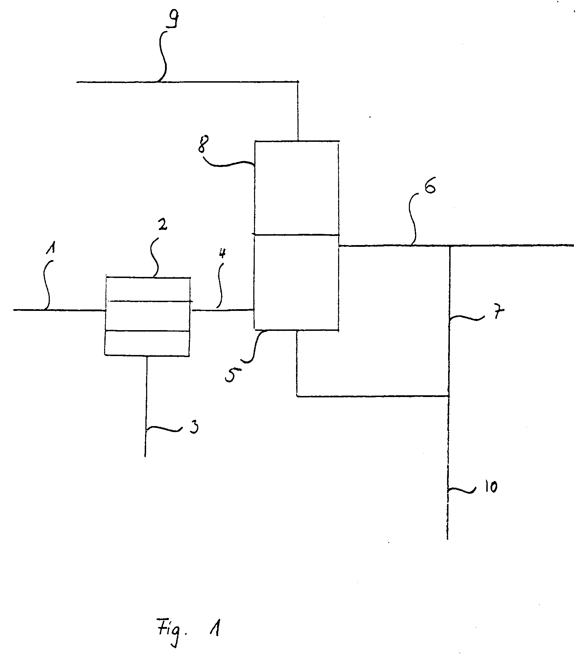

Das Verfahren wird im folgenden im Zusammenhang mit Fig. 1, die eine Vorrichtung zur Durchführung des erfindungsgemäßen Verfahrens wiedergibt, anhand eines konkreten Ausführungsbeispiels weiter erläutert. Das erfindungsgemäße Aufarbeitungsverfahren wird hier im Rahmen der Herstellung von CF₃CHCl₂ aus Tetrachlorethylen und Fluorwasserstoff durchgeführt. Die bei der Umsetzung von Tetrachlorethylen und Fluorwasserstoff erhaltenen flüchtigen Bestandteile (im wesentlichen HCl, HF, organische Verbindungen und Wasser) wurden über die Leitung 1 in einen Phasentrenner 2 eingeleitet und kondensiert. Es bildeten sich zwei Phasen. Die schwerere Phase bestand im wesentlichen aus CF₃CHCl₂, teilfluorierten Verbindungen und Ausgangsverbindung. Diese schwerere Phase wurde über die Leitung 3 aus dem Phasentrenner 2 abgelassen und durch Destillation weiter aufgetrennt.

Die leichtere Phase, im folgenden Fluorwasserstoff-Phase genannt, bestand im wesentlichen aus HF, HCl, Wasser und leichtflüchtigen organischen Verbindungen.

Diese Phase wurde über die Leitung 4 in eine Trocknungskolonne 5 eingespeist. Über die Leitung 6 wurde Oleum (40 Gew.-% SO₃, bezogen auf die als 100 Gew.-% gesetzte H₂SO₄) der Trocknungskolonne 5 zugeführt. Der Kontakt zwischen Oleum und der im Gegenstrom geführten Fluorwasserstoff-Phase wurde durch Füllkörper noch intensiviert. Ein Teil der für die Trocknung verbrauchten Schwefelsäure wurde in die Fluorwasserstoff-Gewinnung aus Flußspat überführt (Leitung 10). Der Rest wurde über die Leitung 7 in die Leitung 6 eingeleitet, dort mit frischem Oleum vermischt und dann in die Trockenkolonne 5 rückgeführt. Der vom Wasser weitgehend befreite Fluorwasserstoff stieg in die Rektifizierkolonne 8 auf und wurde dort von mitgerissener Schwefelsäure und Oleum befreit.

Der rektifizierte Fluorwasserstoff wurde über die Leitung 9 in die Fluorierungsapparatur zurückgeführt.

Vorstehend wurde die Anwendung des erfindungsgemäßen Verfahrens auf die von der organischen Phase abgetrennte Fluorwasserstoff-Phase aus der Herstellung von CF₃CHCl₂ beschrieben. Für den Fachmann ist klar, daß das erfindungsgemäße Aufarbeitungsverfahren auch im Rahmen von anderen Herstellverfahren für Fluor enthaltende Kohlenwasserstoffe eingesetzt werden kann, in welchen Fluorwasserstoff-Phasen anfallen, die Leichtsieder und Wasser enthalten.

## Patentansprüche

1. Verfahren zur Aufarbeitung der Fluorwasserstoff-Phase von Reaktionsgemischen aus Fluorierungsreaktionen, wobei man aus der Reaktionsmischung zwei Phasen bildet, deren eine Phase im wesentlichen aus organischem Reaktionsprodukt besteht und die andere Phase eine Fluorwasserstoff-Phase ist, man die Fluorwasserstoff-Phase von der anderen Phase abtrennt, und man die Fluorwasserstoff-Phase zur Wasserabtrennung bei einer Temperatur von 160 °C bis 200 °C mit Oleum kontaktiert und den resultierenden Fluorwasserstoff in die Fluorierungsreaktion rückführt.

2. Verfahren nach Anspruch 1, dadurch gekennzeichnet, daß die Entwässerung in einer Füllkörperkolonne im Gegenstrom erfolgt.

3. Verfahren nach Anspruch 1 oder 2, dadurch gekennzeichnet, daß das verbrauchte Oleum aufgestärkt und zur Trocknung wiederverwendet wird.

4. Verfahren nach Anspruch 1 oder 2, dadurch gekennzeichnet, daß das verbrauchte Oleum in einem Verfahren zur Herstellung von Fluorwasserstoff durch Umsetzung von Flußspat mit Schwefelsäure verwendet wird.

5. Verfahren nach Anspruch 1, dadurch gekennzeichnet, daß mit Oleum einer Konzentration von 1 bis 40 Gew.-% SO₃, bezogen auf die als 100 Gew.-% gesetzte H₂SO₄, entwässert wird.

6. Verfahren nach Anspruch 1, dadurch gekennzeichnet, daß man den resultierenden Fluorwasserstoff in einer Rektifikationskolonne rektifiziert.

7. Verfahren nach einem der vorhergehenden Ansprüche, dadurch gekennzeichnet, daß die Rektifikationskolonne auf eine Temperatur von 10 bis 20 °C Kopftemperatur und 160 °C bis 200 °C im unteren Bereich temperiert wird.

## Claims

1. A process for working up the hydrogen fluoride phase of reaction mixtures from fluorination reactions, in which two phases are formed from the reaction mixture, one phase of which essentially consists of organic reaction product and the other phase of which is a hydrogen fluoride phase, the hydrogen fluoride phase is separated from the other phase, and the hydrogen fluoride phase is contacted with oleum at a temperature of 160°C to 200°C in order to separate off water, and the resulting hydrogen fluoride is recycled into the fluorination reaction.

2. A process according to Claim 1, characterised in that the dehydration takes place in a packed column in a counter-current.

3. A process according to Claim 1 or 2, characterised in that the spent oleum is fortified and re-used for drying.

4. A process according to Claim 1 or 2, characterised in that the spent oleum is used in a process for producing hydrogen fluoride by reacting fluorite with sulphuric acid.

5. A process according to Claim 1, characterised in that dehydration is performed with oleum having a concentration of 1 to 40% by weight SO₃, relative to the H₂SO₄ set as 100% by weight.

6. A process according to Claim 1, characterised in that the resulting hydrogen fluoride is rectified in a rectifying column.

7. A process according to one of the preceding Claims, characterised in that the temperature of the rectifying column is controlled at 10 to 20°C head temperature and 160°C to 200°C in the lower region.

## Revendications

1. Procédé pour le traitement de la phase d'acide fluorhydrique de mélanges réactionnels issus de réactions de fluoruration, où l'on forme, à partir du mélange réactionnel, deux phases, dont l'une est essentiellement composée d'un produit réactionnel organique, et l'autre est une phase d'acide fluorhydrique, où l'on sépare la phase d'acide fluorhydrique de l'autre phase, et où l'on met en contact la phase d'acide fluorhydrique, pour séparer l'eau, avec de l'oléum à une température de 160 °C à 200 °C, et où l'on remet l'acide fluorhydrique résultant dans la réaction de fluoruration.

2. Procédé selon la revendication 1, caractérisé en ce que la dessiccation est effectuée dans une colonne à corps de remplissage à contre-courant.

3. Procédé selon la revendication 1 ou 2, caractérisé en ce que l'oléum usagé est concentré et réutilisé pour le séchage.

4. Procédé selon la revendication 1 ou 2, caractérisé en ce que l'oléum usagé est utilisé dans un procédé pour la fabrication d'acide fluorhydrique par conversion de la fluorine avec de l'acide sulfurique.

5. Procédé selon la revendication 1, caractérisé en ce que la déshydratation est effectuée avec de l'oléum ayant une concentration de 1 à 40 % en poids de SO_{3,} par rapport au H₂SO₄ dosé à 100 % en poids.

6. Procédé selon la revendication 1, caractérisé en ce que l'on rectifie l'acide fluorhydrique résultant dans une colonne de rectification.

7. Procédé selon l'une des revendications précédentes, caractérisé en ce que la colonne de rectification est réglée à une température de 10 à 20 °C dans la zone supérieure de la colonne et de 160 °C à 200 °C dans la zone inférieure.
